# EUROPEAN PATENT APPLICATION

(11) **EP 2 921 195 A1**
(43) Date of publication of application: **23.09.2015**
(21) Application number: 15156545.4
(22) Date of filing: 25.02.2015
(51) Int. Cl.: A61M 25/09

(54) **Guide wire**

(30) Priority: 26.02.2014 JP 2014035222
(71) Applicant: ASAHI INTECC CO., LTD., Nagoya-shi, Aichi 463-0024 (JP)
(72) Inventor: Furukawa, Muneya, Nagoya-shi, Aichi 463-0024 (JP); Tsuge, Kenta, Nagoya-shi, Aichi 463-0024 (JP)
(74) Representative: Winter, Brandl, Fürniss, Hübner, Röss, Kaiser, Polte - Partnerschaft mbB

(57) **Abstract**

[Problem to be Solved]

To provide a guide wire having a sufficient flexibility, and capable of suppressing a shifting of a bending point from a distal side to a proximal side even when the guide wire collides with a lesion part.

[Solution]

A guide wire 10 comprises a shaft 12 and a first coil body 20 wound around a distal portion of the shaft 12. The first coil body 20 is formed by helically twisting a twisted wire 22 which is formed by twisting a plurality of single strands 21 together, and a joint portion 35 disposed in a intermediate part of the first coil body 20 and impregnated into the twisted wire 22 of the first coil body 20 without contacting the shaft 12, so as to join adjoining turns of the twisted wire 22 with each other.

## Description

### [Technical Field]

The present invention relates to a guide wire used as a medical device to be inserted into a body cavity for medical treatment or examination.

### [Background Art]

Conventionally, various guide wires that guide catheter or the like to be inserted into tubular organs such as blood vessels, digestive tracts, and ureters, or in body tissues have been proposed for medical treatment or examination.

For example, Patent Literature 1 discloses a guide wire comprising a shaft whose diameter gradually becomes narrower as it goes toward a distal end side of the shaft and a coil wound around a distal portion of the shaft, in which an intermediate part of the coil is fixed to the shaft by a fixing material.

### [Citation List]

### [Patent Literature]

[Patent Literature 1]
Japanese Laid-Open Patent Publication No. 2007-90097

### SUMMARY OF INVENTION

### [Technical Problem]

For example, when using a guide wire inside of blood vessels in a lower limb, a sufficient flexibility is required at a distal portion of the guide wire in order to ensure excellent follow-up capability and selectivity, since the blood vessels are three-dimensionally and intricately windings. Moreover, for example, if a distal end of the guide wire collides with a hard lesion part during a use of the guide wire in the lower limb, the distal portion may be bent, and if the guide wire is continuously used and inserted further with this state, the bending condition may possibly be expanded. That is, a bending point of the guide wire may shift from the distal portion to a proximal portion and may even reach a large diameter portion of the shaft. As a result, the bending point (the large diameter portion of the shaft) may plastically deform, which makes it hard to recover the original shape of the distal portion by hand.

In such a case, if a rigidity of a substantially intermediate portion of the guide wire is locally enhanced, the guide wire can bend at such a high rigidity portion when the distal end of the guide wire collides with a hard lesion part. As a result, a collision stress is alleviated, which may suppress above-mentioned "shifting of the bending point from the distal portion to the proximal portion", and further, suppress a plastic deformation of the large diameter portion of the shaft.

However, in the above-mentioned Patent Literature 1, since the intermediate part of the coil is fixed to the shaft by the fixing material, a rigidity at a fixing portion is locally enhanced, while a flexibility of the guide wire is deteriorated because the shaft is restrained by the fixing material. On the other hand, although adjusting the quantity of the fixing material appropriately may be possible in order to ensure the flexibility of the distal portion of the guide wire, such an adjustment may be troublesome and may not provide sufficient rigidity for the fixing portion. For this reason, if the distal end of the guide wire collides with a lesion part, there is a possibility of shifting of the bending point from the distal portion to the proximal portion. That is, the guide wire disclosed in the above-mentioned Patent Literature 1 still has room for improvement in that the sufficient flexibility is ensured as well as the rigidity which would be able to suppress the shifting of the bending point from the distal portion to the proximal portion even when the guide wire collides with a lesion part.

From the viewpoint of the above circumstances, an object of the present invention is to provide a guide wire having a sufficient flexibility and rigidity that would be able to suppress the shifting of a bending point from the distal portion to the proximal portion even when the guide wire collides with a lesion part.

### [Solution to Problem]

In order to solve the above-mentioned problems, the guide wire of one aspect of the present invention includes following features.

A guide wire of first aspect of the present invention comprises a shaft and a first coil body comprising a plurality of turns and wound around a distal portion of the shaft, in which the first coil body is formed by helically twisting at least one twisted wire which is formed by twisting a plurality of single strands together, and a joint portion impregnated into the at least one twisted wire is disposed in an intermediate part located between a distal end and a proximal end of the first coil body without contacting the shaft, so as to join adjoining turns of the first coil body.

Second aspect of the present invention is the guide wire of the first aspect, wherein a pitch of the first coil body increases from a proximal end to the distal end.

Third aspect of the present invention is the guide wire as described in the first or second aspect, wherein the first coil body of the guide wire is formed by helically twisting a plurality of twisted wires.

Fourth aspect of the present invention is the guide wire described in any one of the first to third aspect, wherein a second coil body is disposed inside the first coil body and is wound around a distal portion of the shaft, and the joint portion joins the first coil body and the second coil body without contacting the shaft.

Fifth aspect of the present invention is the guide wire as described in the fourth aspect, wherein the second coil body is formed by helically twisting at least one twisted wire which is formed by twisting a plurality of single strands together.

Sixth aspect of the present invention is the guide wire as described in the fifth aspect, wherein the second coil body of the guide wire is formed by helically twisting a plurality of twisted wires.

Seventh aspect of the present invention is the guide wire as described in the fourth aspect, wherein the second coil body is formed by helically twisting a single strand.

Eighth aspect of the present invention is the guide wire as described in the fourth aspect, wherein the second coil body is formed by twisting a plurality of single strands together.

Ninth aspect of the present invention is the guide wire as described in any one of the first to eighth aspect, wherein the joint portion is formed by impregnating resin material into the at least one twisted wire.

### [Advantageous Effects of Invention]

The guide wire of the first aspect of the present invention comprises a shaft and a first coil body comprising a plurality of turns and wound around the distal portion of the shaft. The first coil body is formed by helically twisting at least one twisted wire which is formed by twisting a plurality of single strands together. A joint portion impregnated into the at least one twisted wire is disposed in the intermediate part of the first coil body without contacting the shaft, so as to join the adjoining twisted wires.

When the joint portion is disposed on the coil body formed by twisting the at least one twisted wire (the twisted wire is formed by twisting a plurality of single strands together), a material forming the joint portion (hereinafter referred to as joint material) permeates into gaps between the single strands of each twisted wire preferentially along the longitudinal direction due to the capillary phenomenon, thus, the rigidity at the joint portion is enhanced. On the contrary, it becomes relatively difficult for the joint material to permeate in the direction orthogonal to the longitudinal direction (the direction heading toward the shaft), so that the joint portion is not connected to the shaft, which prevents the shaft from being restrained by the joint portion.

Consequently, the flexibility at the distal portion of the guide wire is ensured without having a complicated process as adjusting the appropriate quantity of the joint material, and the sufficient rigidity is also ensured by allowing the joint material to be inserted between the single strands along the longitudinal direction due to the capillary phenomenon and jointing the adjoining twisted wires each other.

Therefore, the guide wire of the first aspect ensures excellent follow-up capability and selectivity in blood vessels that are intricately and three-dimensionally winding in the lower limb, and when the distal end of the guide wire collides with a hard lesion part, a collision stress is concentrated on the intermediate part of the coil body (a part at which the rigidity is enhanced by the joint portion), and then the guide wire bends at the intermediate part in substantially V-shape to diminish the stress, so that the stress becomes less transmittable to a proximal side beyond the intermediate part. As a result, even when the distal end of the guide wire collides with a hard lesion part, a bending point of the guide wire does not shift to a large diameter section of the shaft and the problem of plastic deformation on the shaft is suppressed, so that the guide wire can be used continuously.

The first coil body of the guide wire in the third aspect is formed by helically twisting a plurality of twisted wires (each twisted wire is formed by twisting a plurality of single strands together). In the constitution, a gap between single strands becomes smaller by allowing the twisted wires to be closely contacted. Moreover, in comparison to the first aspect described above, the permeation of the joint material into gaps between single strands along the longitudinal direction is further facilitated, thus, the rigidity at the joint portion is reliably enhanced. On the contrary, it becomes relatively difficult for the joint material to permeate in the direction orthogonal to the longitudinal direction (the direction heading toward the shaft), so that the joint portion is not connected to the shaft, which prevents the shaft from being restrained by the joint portion.

Thereby, the guide wire of the third aspect ensures excellent follow-up capability and selectivity in blood vessels that are three-dimensionally and intricately winding in the lower limb, and reliably can allow collision stress to concentrate on the intermediate part (a part at which the rigidity is enhanced by the joint portion) of the coil body even if the distal end of the guide wire collides with a hard lesion part, and therefore, the stress is alleviated by allowing the guide wire to bend in substantially V-shape. Then, the stress becomes less transmittable to the proximal side beyond the intermediate part of the coil body, so that the problem of causing a plastic deformation on the shaft can be suppressed reliably.

A guide wire in the fourth aspect comprises a second coil body disposed inside the first coil body, wherein the joint portion joins the first coil body and the second coil body without contacting the shaft. In the constitution, when the joint portion is provided, the permeation of the joint material into the gaps between single strands constituting the first coil body is further facilitated due to the capillary phenomenon as described above, while the permeation of the joint material in the direction orthogonal to the longitudinal direction is blocked by the second coil body, so that the permeation of the joint material to the shaft is effectively suppressed.

Therefore, the guide wire of the fourth aspect can ensure the rigidity at the joint portion, and suppress the permeation of the joint material to the shaft, which eliminates the possibility of the shaft being restrained by the joint portion, thus, ensuring the sufficient flexibility.

That is, as described above, the guide wire of thefourth aspect also can ensure excellent follow-up capability and selectivity in the blood vessels that are three-dimensionally and intricately winding in the lower limb, and even if the distal end of the guide wire collides with a hard lesion part, the guide wire can alleviate the collision stress by allowing the stress to easily concentrate on the intermediate part of the coil body (a part at which the rigidity is enhanced by the joint portion) and allowing the guide wire itself to bend in substantially V-shape. Then, the stress becomes less transmittable to the proximal side beyond the intermediate part of the coil body, so that the problem of causing a plastic deformation on the shaft can be suppressed effectively.

The second coil body of the guide wire in the fith aspect comprises at least one twisted wires (each twisted wire is formed by helically twisting a plurality of single strands together). When the joint portion is disposed in the intermediate part of the first coil body, the joint material permeates, also in the second coil body, into the gaps between single strands preferentially along the longitudinal direction due to the capillary phenomenon, thus, the rigidity is enhanced. That is, in the guide wire of the fifth aspect, the joint material permeates in each of the gaps between single strands constituting the first coil body and the second coil body, respectively, along the longitudinal direction. On the other hand, the permeation of the joint material in the direction orthogonal to the longitudinal direction is blocked by the second coil body, so that the permeation of the joint portion to the shaft can be reliably suppressed.

Consequently, the guide wire in the fifth aspect can ensure a sufficient rigidity at the joint portion formed by a joint material permeating along the longitudinal direction of both the first coil body and the second coil body, and can ensure a sufficient flexibility without allowing the joint portion to connect to the shaft.

That is, as described above, the guide wire of the fifth aspect also can ensure excellent follow-up capability and selectivity in the blood vessels that are three-dimensionally and intricately winding in the lower limb, and even if the distal end of the guide wire collides with a hard lesion part, the guide wire can allow the collision stress to reliably concentrate on the intermediate part of the coil body (a part at which the rigidity is enhanced by the joint portion). Then, as the stress is alleviated by the guide wire bending in substantially V-shape at the intermediate part where the rigidity is locally enhanced, the stress becomes less transmittable to the proximal side beyond the intermediate part of the coil body, so that the problem of causing a plastic deformation on the shaft can be suppressed more reliably.

A second coil body of the guide wire in the sixth aspect is formed by helically twisting a plurality of twisted wires (each twisted wire is formed by twisting a plurality of the single strands together). For the second coil body having such a constitution, gaps between single strands becomes smaller by allowing the twisted wires to be closely contacted each other. As a result, when the joint portion is disposed at the intermediate part of the first coil body, the permeation of the joint material into gaps between single strands that form the second coil body along the longitudinal direction is further facilitated compared to the fifth aspect described above, thus, the rigidity at the joint portion is enhanced. On the other hand, the permeation of the joint portion in the direction orthogonal to the longitudinal direction is blocked by the second coil body, so that the permeation of the joint material to the shaft can be reliably suppressed.

Therefore, the guide wire of the sixth aspect can reliably ensure the rigidity at the joint portion, and eliminate the possibility of the shaft being restrained by the joint portion, thus, the sufficient flexibility is ensured. That is, as described above, the guide wire of the sixth aspect also can ensure excellent follow-up capability and selectivity in the blood vessels that are three-dimensionally and intricately winding in the lower limb, and even if the distal end of the guide wire collides with a hard lesion part, the guide wire can allow the collision stress to reliably concentrate on the intermediate part of the coil body (a part at which the rigidity is enhanced by the joint portion). Then, as the stress is alleviated by the guide wire bending in substantially V-shape at the intermediate part where the rigidity is locally enhanced, the stress becomes less transmittable to the proximal side beyond the intermediate part of the coil body, so that the problem of causing a plastic deformation on the shaft can be suppressed more reliably.

The seventh and eighth aspects relate to specific embodiments of the guide wire in accordance with the fourth aspect, so that the same advantageous effects can be achieved as described with the above aspects.

### BRIEF DESCRIPTION OF DRAWINGS

FIG.1 is a partial enlarged cross-sectional view that illustrates a guide wire in a first embodiment of the present invention.
FIG. 2 is a perspective view that illustrates a twisted wire for constituting a first coil body in the first embodiment of the present invention.
FIG.3 is an enlarged cross-sectional view that illustrates a joint portion in the first embodiment of the present invention.
FIG.4 is a partial enlarged cross-sectional view that illustrates the guide wire in the first embodiment of the present invention in a bending condition.
FIG.5 is a partial enlarged cross-sectional view of another example that illustrates the guide wire in the first embodiment of the present invention.
FIG.6 is a partial enlarged cross-sectional view that illustrates a guide wire in a second embodiment of the present invention.
FIG.7 is a cross-sectional view taken from line A-A of FIG.6.
FIG.8 is an enlarged cross-sectional view that illustrates a joint portion in the second embodiment of the present invention.
FIG.9 is a partial enlarged cross-sectional view that illustrates the guide wire in the second embodiment of the present invention in a bending condition.
FIG.10 is a partial enlarged cross-sectional view that illustrates a guide wire in a third embodiment of the present invention.
FIG.11 is a partial enlarged cross-sectional view that illustrates the guide wire in the third embodiment of the present invention in a bending condition.
FIG.12 is a partial enlarged cross-sectional view that illustrates a guide wire in a fourth embodiment of the present invention.
FIG. 13 is a perspective view that illustrates a second coil body in the fourth embodiment of the present invention.
FIG.14 is an enlarged cross-sectional view that illustrates a joint portion in the fourth embodiment of the present invention.
FIG.15 is a partial enlarged cross-sectional view that illustrates the guide wire in the fourth embodiment of the present invention in a bending condition.
FIG.16 is a partial enlarged cross-sectional view that illustrates a guide wire in a fifth embodiment of the present invention.
FIG. 17 is a cross-sectional view taken from line B-B of FIG. 16.
FIG.18 is a partial enlarged cross-sectional view that illustrates the guide wire in the fifth embodiment of the present invention in a bending condition.

### DESCRIPTION OF EMBODIMENTS

The guide wire of the present invention is explained according to embodiments illustrated in drawings.

### [First embodiment]

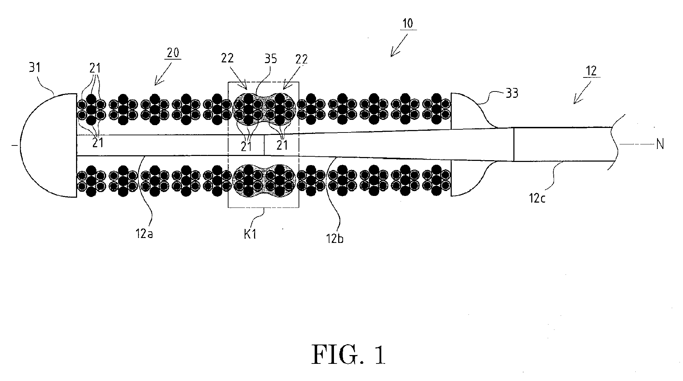

FIG.1 is a partial enlarged cross-sectional view that illustrates a first embodiment of the guide wire of the present invention. In FIG. 1, the distal side from which the guide wire is inserted in a body is shown in the left, and the proximal side by which a doctor or other technician manipulates is shown in the right. In the figure, the guide wire is schematically illustrated, so that the proportion of sizes may be different from actual ones including the cross-sectional shape of the coil body that is formed by a twisted wire.

For example, a guide wire 10 as illustrated in FIG.1 is used for medical treatment of lower limb blood vessels according to the Cross Over method. The guide wire 10 comprises a shaft 12 and a first coil body 20 covering the periphery of a distal portion of the shaft 12.

Firstly, the shaft 12 is explained. The shaft 12 includes a thin diameter section 12a, a taper section 12b and a large diameter section 12c in this order from the distal side to the proximal side. The thin diameter section 12a is disposed at the most distal side of the shaft 12 and the most flexible portion of the shaft 12. The thin diameter section 12a is pressed and formed in a tabular shape. The taper section 12b has a taper shape with a round cross-section, and is narrowing its diameter as it goes toward the distal side. The large diameter section 12c has a larger diameter than that of the thin diameter section 12a.

Although not limited to these, a material used for forming the shaft 12, for example, includes: stainless steel (SUS304); superelastic alloy such as Ni-Ti alloy; piano wire; and cobalt alloy.

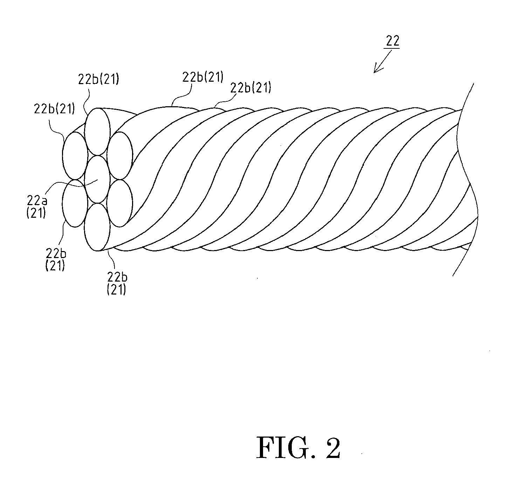

Next, the first coil body 20 is explained. The first coil body 20 of the embodiment is formed by helically twisting a twisted wire 22 (see FIG.2). The twisted wire 22 is formed by twisting a plurality of single strands 21 together. The twisted wire 22 includes a core strand 22a and six side strands 22b twisted about the periphery of the core strand 22a for covering the core strand 22a. As shown in FIG.1, in this embodiment, a pitch of a helix of the first coil body 20 is evenly arranged in the longitudinal direction "N".

Although not particularly limited to these, for example, a material used for forming the core strand 22a and the side strand 22b includes: stainless steel such as martensitic stainless, ferritic stainless, austenitic stainless, austenitic/ferrite two phase stainless, or precipitation-hardening stainless; superelastic alloy such as Ni-Ti alloy; roentgenopaque metal such as platinum, gold, tungsten, tantalum, iridium, or alloys thereof.

The distal end of the first coil body 20 is fixed to the distal end of the shaft 12 by a distal fixing portion 31. The proximal end of the first coil body 20 is fixed to the shaft 12 by a proximal fixing portion 33. Moreover, a joint portion 35 is disposed in the intermediate part of the first coil body 20. Although not particularly limited to these, a material used for forming the distal fixing portion 31, the proximal fixing portion 33, or the joint portion 35, for example, includes brazing metal such as Sn-Pb alloy, Pb-Ag alloy, Sn-Ag alloy, or Au-Sn alloy.

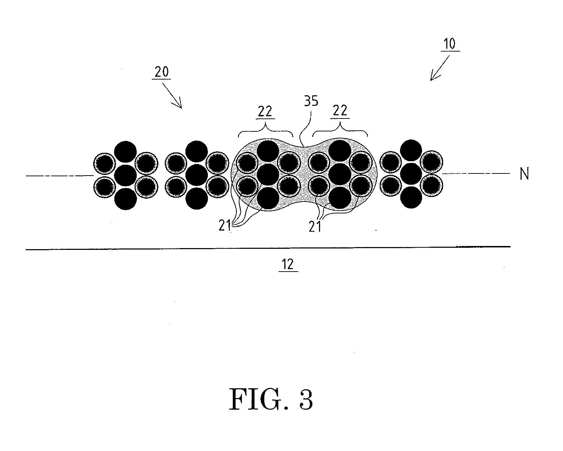

As shown in FIGS.1 and 3, the joint portion 35 is impregnated into the twisted wire 22, i.e. entered into gaps between the single strands 21 that form the first coil body 20 (the twisted wire 22), and joins at least one pair of adjoining turns of the first coil body 20, without the joint portion 35 being connected to the shaft 12.

By disposing the joint portion 35 in the first coil body 20 formed by twisting the twisted wire 22 (the twisted wire 22 is formed by twisting a plurality of single strands 21 together), the joint material for forming the joint portion 35 permeates into gaps between single strands 21 preferentially along the longitudinal direction "N" due to the capillary phenomenon, thus, enhancing the rigidity at the joint portion. On the contrary, it becomes relatively difficult for the joint portion 35 to permeate in the direction orthogonal to the longitudinal direction "N" (the direction heading toward the shaft 12), so that the joint portion 35 is not connected to the shaft 12, which prevents the shaft 12 from being restrained by the joint portion 35.

Consequently, the flexibility at the distal portion of the guide wire 10 is ensured without having such a troublesome process as adjusting the appropriate quantity of the joint portion 35, and a sufficient rigidity is also ensured by allowing the joint portion 35 to be impregnated into the twisted wire 22, i.e. entered into gaps between the single strands 21 due to the capillary phenomenon for jointing the adjoining twisted wires 22.

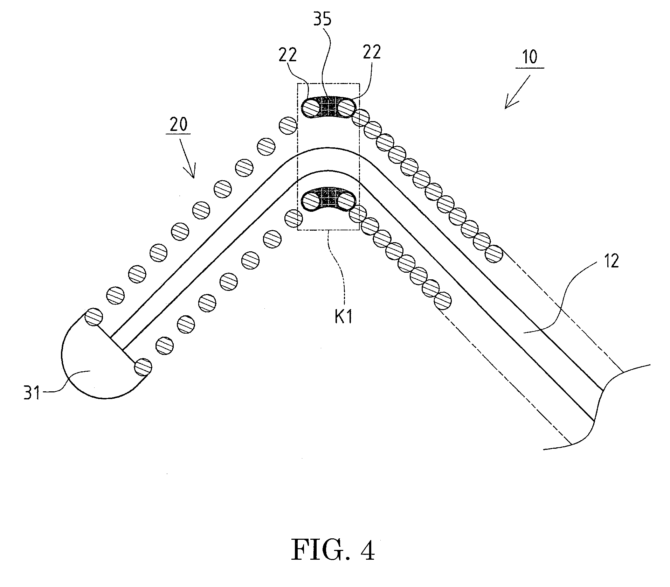

Therefore, the guide wire 10 of the embodiment can ensure excellent follow-up capability and selectivity in blood vessels that are intricately and three-dimensionally winding in the lower limb, and even when the distal end of the guide wire collides with a hard lesion part, the collision stress is concentrated on the intermediate part of the first coil body 20 (the part K1 at which the rigidity is enhanced by the joint portion 35), and then the stress is alleviated by the guide wire 10 being bent in substantially V-shape as in FIG. 4, so that the stress becomes less transmittable to the proximal side beyond the intermediate part of the first coil body 20. As a result, even the distal end of the guide wire 10 collides with a hard lesion part, the problem of plastic deformation of the shaft 12 by shifting the bending point to the large diameter section 12c is suppressed, so that the guide wire 10 can be used continuously.

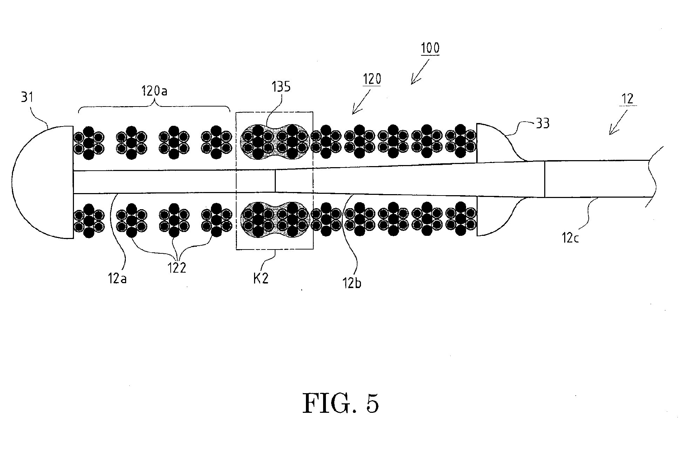

Although the pitch of the helix of the first coil body 20 of the embodiment is evenly arranged in the longitudinal direction "N", the helical condition of the first coil body is not limited to this. That is, as shown in FIG.5, a sparse twisting section 120a having a relatively larger gap between adjoining twisted wires 122 may be disposed at the distal side of the first coil body 120 (distal side beyond the joint portion 135).

For such a guide wire 100, the flexibility at the distal portion is further improved, so that excellent follow-up capability and selectivity in blood vessels are ensured easily, and as it goes from the distal end to the proximal portion, the rigidity of the guide wire 10 is sharply enhanced owing to the joint portion 135. As a result, when the distal end of the guide wire 100 collides with a hard lesion part, the collision stress is likely to concentrate on the intermediate part of the first coil body 120 (the part K2 at which the rigidity is enhanced by the joint portion 135), the guide wide is bent in substantially V-shape, and the stress can be alleviated effectively.

### [Second embodiment]

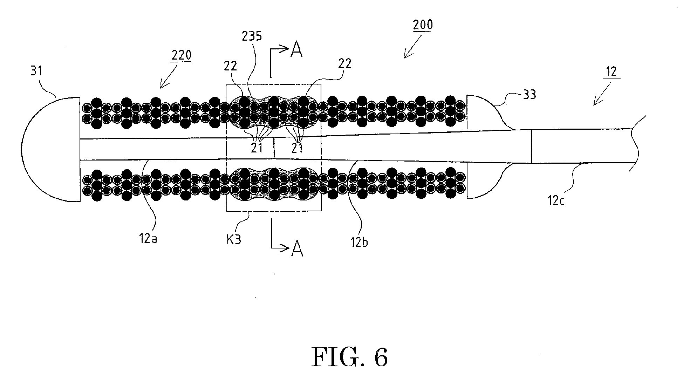

FIG.6 is a partial enlarged cross-sectional view that illustrates a second embodiment of the guide wire of the present invention. In FIG. 6, the distal side from which the guide wire is inserted in the body is shown in the left, and the proximal side by which a doctor or other technician manipulates is shown in the right. In the figure, the guide wire is schematically illustrated, so that the proportion of sizes may be different from actual ones including the cross-sectional shape of the coil body that is formed by the twisted wire.

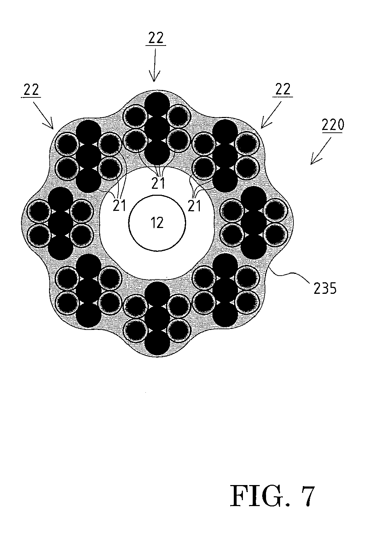

In the first embodiment described above, the first coil body formed by helically twisting the twisted wire is employed. In a guide wire 200 of the second embodiment as shown in FIG.7, a first coil body 220 formed by helically twisting a plurality of twisted wires 22 of the first embodiment (eight twisted wires are used in this embodiment) is employed.

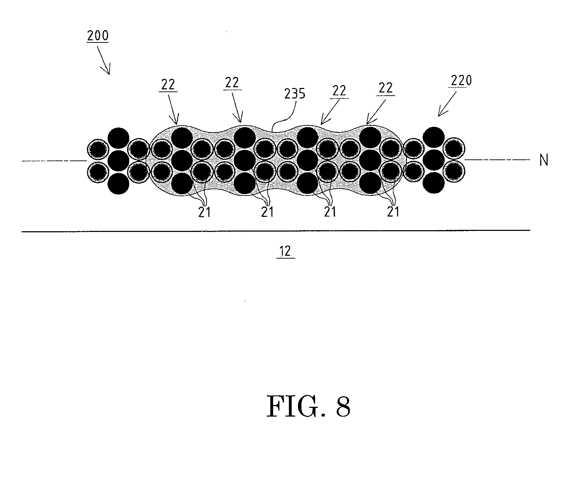

As for the first coil body 220 which is formed by helically twisting a plurality of twisted wires 22, the twisted wires 22 are closely contacted each other and the gaps between the single strands 21 become further small. As a result, in comparison to the first embodiment described above, the permeation of the joint material (the joint portion 235) into gaps between single strands 21 along the longitudinal direction "N" is further facilitated (see FIG.8), thus, reliably enhancing the rigidity at the joint portion. On the contrary, it becomes difficult for the joint material (the joint portion 235) to permeate in the direction orthogonal to the longitudinal direction "N" (the direction heading toward the shaft 12), so that the joint portion 235 is not connected to the shaft 12, which prevents the shaft 12 from being restrained by the joint portion 235.

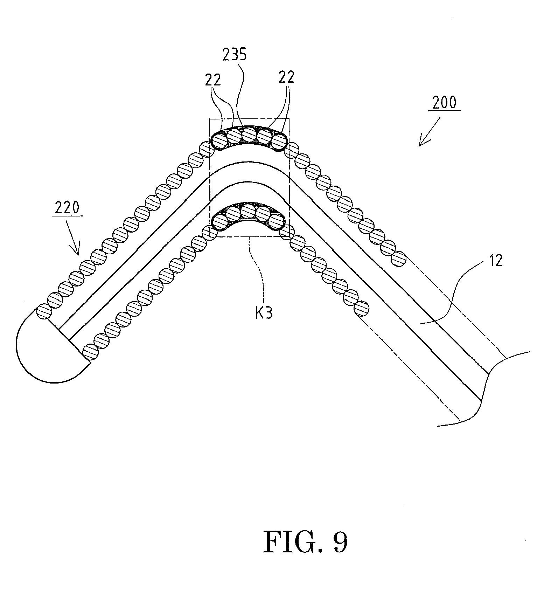

According to the second embodiment, the guide wire 200 can ensure excellent follow-up capability and selectivity in blood vessels that are three-dimensionally and intricately winding in the lower limb, and reliably can allow collision stress to concentrate on the intermediate part of the first coil body 220 (the part K3 at which the rigidity is enhanced by the joint portion 235) even if the distal end of the guide wire collides with a hard lesion part. As a result, the stress is alleviated by allowing the guide wire 200 to bend in substantially V-shape as shown in FIG. 9 at the part K3 at which the rigidity is enhanced by the joint portion 235. Then, as the stress becomes less transmittable to the proximal side beyond the intermediate part of the first coil body 220, the problem of causing a plastic deformation on the shaft 12 can be reliably suppressed.

### [Third embodiment]

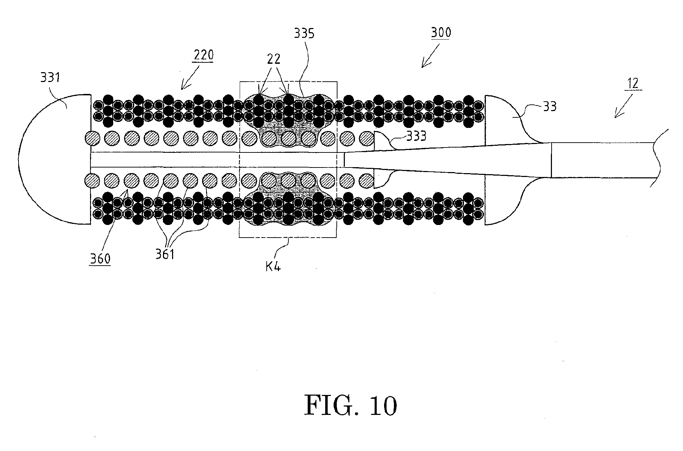

FIG.10 is a partial enlarged cross-sectional view that illustrates a third embodiment of the guide wire of the present invention. In FIG. 10, the distal side from which the guide wire is inserted in the body is shown in the left, and the proximal side by which a doctor or other technician manipulates is shown in the right. In the figure, the guide wire is schematically illustrated, so that the proportion of sizes may be different from actual ones including the cross-sectional shape of the coil body that is formed by the twisted wire.

A guide wire 300 of this embodiment includes a second coil body 360 disposed inside of the first coil body 220 which is the same as that in the second embodiment. The second coil body 360 in this embodiment is a single strand coil formed by helically twisting a single strand 361.

Although not limited to these, the second coil body 360 may be formed by a radiopaque strand or a radiolucent strand. Although not particularly limited to these, a material used for the radiopaque strand, for example, includes gold, platinum, tungsten, or an alloy having these elements (e.g., platinum-nickel alloy). Although not particularly limited to these, a material used for the radiolucent strand, for example, includes stainless steel (SUS304, SUS316, or the like), superelastic alloy such as Ni-Ti alloy, and piano wire.

The distal end of the second coil body 360 is connected to the distal end of the shaft 12 by a distal fixing portion 331. The proximal end of the second coil body 360 is connected to the shaft 12 by a proximal fixing portion 333. Although not particularly limited to these, a material used for forming the proximal fixing portion 333, for example, includes brazing metals such as Sn-Pb alloy, Pb-Ag alloy, Sn-Ag alloy, or Au-Sn alloy.

A joint portion 335 disposed in the intermediate part of the first coil body 220 joins the first coil body 220 and the second coil body 360, without contacting the shaft 12. In this embodiment, as described above, the permeation of the joint material (the joint portion 335) into gaps between single strands 21 that constitute the first coil body 220 is facilitated due to the capillary phenomenon by disposing the joint portion 335, while the permeation of the joint material (the joint portion 335) to the shaft 12 can be suppressed easily by the second coil body 360 which is blocking the permeation of the joint portion 335 in the direction orthogonal to the longitudinal direction "N".

Therefore, the guide wire 300 of the embodiment can ensure the rigidity at the joint portion, and suppress the permeation of the joint material to the shaft 12, which eliminates the possibility of the shaft 12 being restrained by the joint portion 335, thus, ensuring a sufficient flexibility.

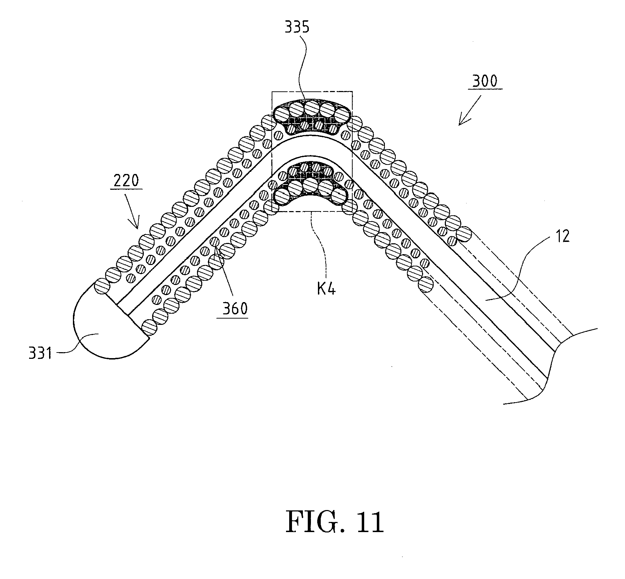

That is, as described above, the guide wire 300 of the embodiment also can ensure excellent follow-up capability and selectivity in blood vessels that are three-dimensionally and intricately winding in the lower limb, and even if the distal end of the guide wire collides with a hard lesion part, the guide wire can alleviate the collision stress by allowing the stress to easily concentrate on the intermediate part of the coil body 220 (the part K4 at which the rigidity is enhanced by the joint portion 335) and allowing the guide wire itself to bend in substantially V-shape (see FIG.11). Then, the stress becomes less transmittable to the proximal side beyond the intermediate part of the coil body 220, so that the problem of causing a plastic deformation on the shaft 12 can be suppressed effectively.

In this embodiment, an example using the first coil body same as that in the second embodiment is described. However, the first coil body of the first embodiment may be employed. Also in this case, the rigidity of desired portion can be enhanced easily by allowing the joint material to preferentially permeate in the longitudinal direction due to the capillary phenomenon.

### [Fourth embodiment]

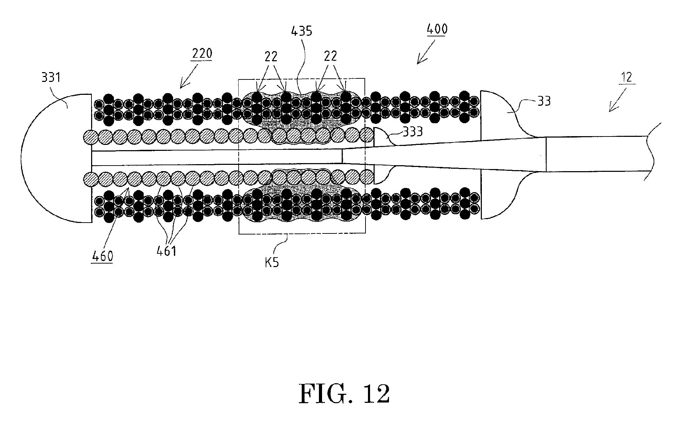

FIG.12 is a partial enlarged cross-sectional view that illustrates a fourth embodiment of the guide wire of the present invention. In FIG. 12, the distal side from which the guide wire is inserted in the body is shown in the left, and the proximal side by which a doctor or other technician manipulates is shown in the right. In the figure, the guide wire is schematically illustrated, so that the proportion of sizes may be different from actual ones including the cross-sectional shape of the coil body that is formed by the twisted wire.

In the above third embodiment, the single strand coil body formed by helically twisting a single strand is employed as the second coil body. In the guide wire 400, a second coil body 460 is formed by twisting a plurality of single strands 461 (e.g, ten single strands 461 are used in this embodiment) .

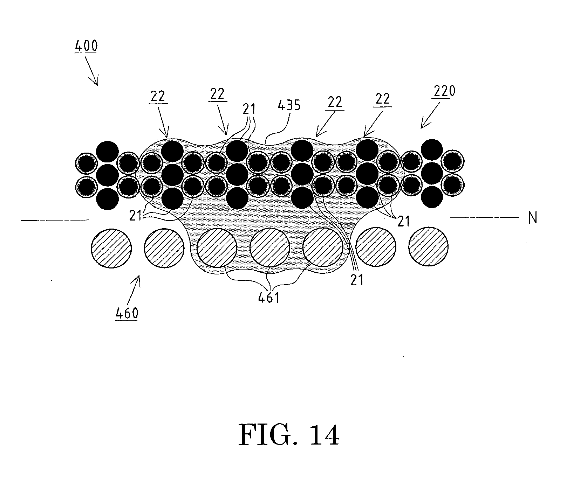

In this embodiment as shown in FIG.14, by disposing a joint portion 435 in the intermediate part of the first coil body 220, also in the second coil body 460 formed by twisting a plurality of single strands 461 together, the joint material (the joint portion 435) permeates into gaps between single strands 461 preferentially along the longitudinal direction "N" due to the capillary phenomenon, so that the rigidity is enhanced. That is, in the guide wire 400 of the embodiment, the joint material (the joint portion 435) permeates in gaps between single strands 21 that form the first coil body 220 and gaps between single strands 461 that form the second coil body 460, along the longitudinal direction "N". On the other hand, the permeation of the joint material (the joint portion 435) in the direction orthogonal to the longitudinal direction "N" is blocked by the second coil body 460, so that the permeation of the joint material (the joint portion 435) to the shaft 12 can be easily suppressed.

Therefore, in the guide wire 400 of the fourth embodiment, the joint portion 435, disposed by permeating in the longitudinal direction "N" with respect to the first coil body 220 and the second coil body 460, can ensure a sufficient rigidity as well as a sufficient flexibility without the shaft 12 being restrained by the joint portion 435.

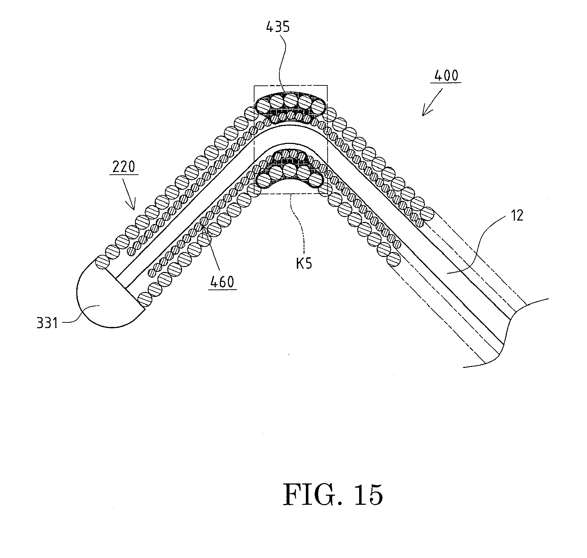

That is, as described above, the guide wire 400 of the embodiment also can ensure excellent follow-up capability and selectivity in blood vessels that are three-dimensionally and intricately winding in the lower limb, and even if the distal end of the guide wire collides with a hard lesion part, the guide wire can alleviate the collision stress by allowing the stress to reliably concentrate on the intermediate part of the coil body 220 (the part K5 at which the rigidity is enhanced by the joint portion 435) and by allowing the guide wire itself to bend in substantially V-shape (see FIG.15). Then, the stress becomes less transmittable to the proximal side beyond the intermediate part of the coil body 220, so that the problem of causing a plastic deformation on the shaft 12 can be suppressed reliably.

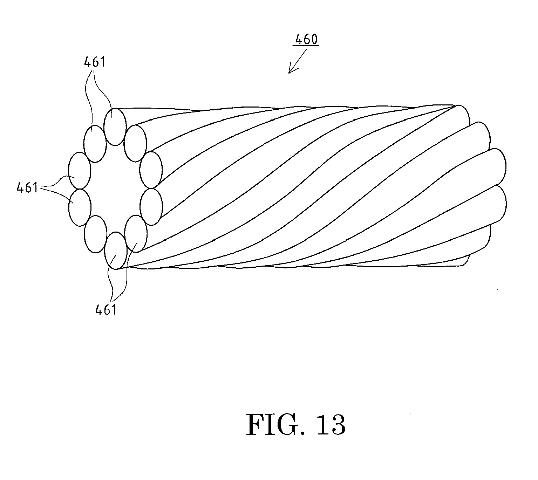

In this embodiment as shown in FIG.13, the second coil body 460 in a hollow shape formed by twisting a twisted wire which is formed by twisting a plurality of single strands 461 together is used. However, the constitution of the second coil body is not limited to this. That is, the second coil body formed by helically twisting the twisted wire containing the core strand and the side strands wound about and covering the periphery of the core strand as shown in FIG. 2 may be used. Also in this case, as with the fourth embodiment, the rigidity of desired portion can be enhanced easily by allowing the joint material to preferentially permeate along the longitudinal direction due to the capillary phenomenon.

### [Fifth embodiment]

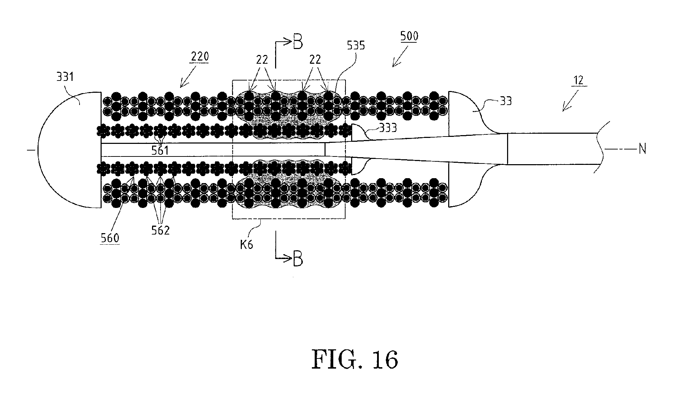

FIG.16 is a partial enlarged cross-sectional view that illustrates a fifth embodiment of the guide wire of the present invention. In FIG.16, the distal side from which the guide wire is inserted in the body is shown in the left, and the proximal side by which a doctor or other technician manipulates is shown in the right. In the figure, the guide wire is schematically illustrated, so that the proportion of sizes may be different from actual ones including the cross-sectional shape of the coil body that is formed by the twisted wire.

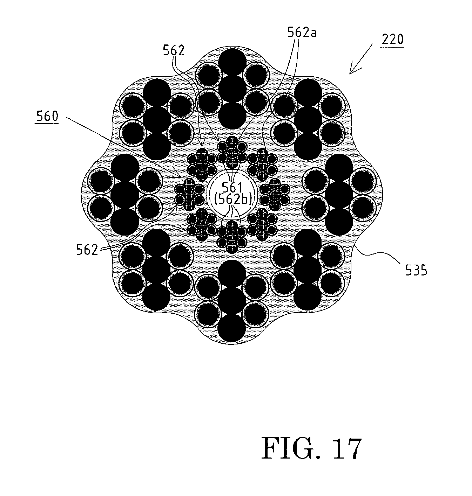

As shown in FIGS. 16 and 17, a second coil body 560 of the guide wire 500 in this embodiment is formed by helically twisting a plurality of twisted wires 562 (eight twisted wires are used in this embodiment), wherein each twisted wire 562 is formed by twisting a plurality of single strands 561 together. More specifically, as shown in FIG. 17, the second coil body 560 is formed by helically twisting eight twisted wires 562, wherein each twisted wire 562 includes a core strand 562a and six side strands 562b wound about the periphery of the core strand 562a for covering the periphery.

Although not particularly limited to these, for example, a material used for forming the core strand 562a and side strand 562b for the second coil body 560 includes: stainless steel such as martensitic stainless, ferritic stainless, austenitic stainless, austenitic/ferrite two phase stainless, or precipitation-hardening stainless; superelastic alloy such as Ni-Ti alloy; roentgenopaque metal such as platinum, gold, tungsten, tantalum, iridium, or alloys thereof.

As shown in this embodiment, for the second coil body 560 formed by helically twisting a plurality of twisted wires 562 (each twisted wire 562 is formed by twisting a plurality of single strands 561 together), the gaps between the single strands 561 become more small by allowing the twisted wires 562 to be more closely contacted each other. As a result, by disposing the joint portion 535 in the intermediate part of the first coil body 220, the permeation of the joint material (the joint portion 535) along the longitudinal direction "N" into gaps between single strands 561 that form the second coil body 560 is further facilitated compared to the fourth embodiment described above, thus, enhancing the rigidity at the joint portion. On the other hand, the permeation of the joint material (the joint portion 535) in the direction orthogonal to the longitudinal direction "N" is blocked by the second coil body 560, so that the permeation of the joint material (the joint portion 535) to the shaft 12 can be reliably suppressed.

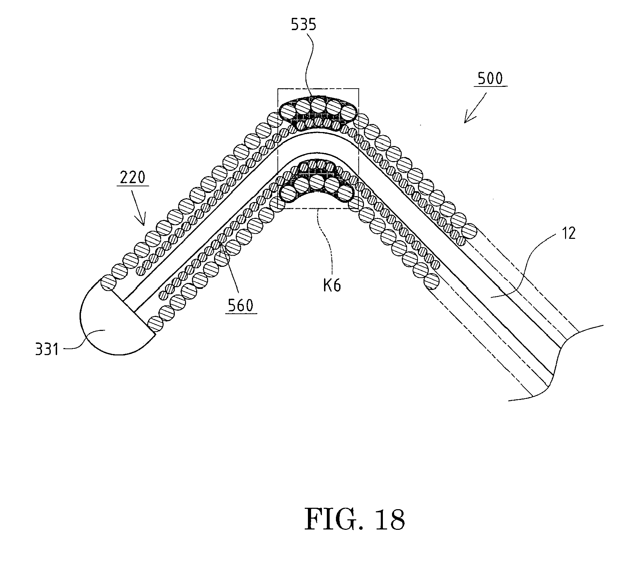

Therefore, the guide wire 500 of the embodiment can reliably ensure the rigidity at the joint portion, and eliminate the possibility of the shaft 12 being restrained by the joint portion 535, thus, ensuring a sufficient flexibility. That is, as described above, the guide wire 500 of the embodiment also can ensure excellent follow-up capability and selectivity in blood vessels that are three-dimensionally and intricately winding in the lower limb, and even if the distal end of the guide wire collides with a hard lesion part, the guide wire can alleviate the collision stress by allowing the stress to further reliably concentrate on the intermediate part of the coil body 220 (the part K6 at which the rigidity is enhanced by the joint portion 535) and by allowing the guide wire 500 itself to bend in substantially V-shape (see FIG.18). Then, the stress becomes less transmittable to the proximal side beyond the intermediate part of the coil body 220, so that the problem of causing a plastic deformation on the shaft 12 can be suppressed more reliably.

### [Reference Signs List]

10, 100, 200,300,400,500 ... Guide wire
12 ... Shaft
20,120, 220 ... First coil body
21 ... Single strand constituting the first coil body
22 ... Twisted wire constituting the first coil body
35, 135, 235,335,435,535 ... Joint portion
360, 460, 560 ... Second coil body
361, 461, 561 ... Single strand constituting the second coil body
562 ... Twisted wire constituting the second coil body

## Claims

1. A guide wire (10, 100, 200, 300, 400, 500) comprising a shaft (12) and a first coil body (20, 120, 220) comprising a plurality of turns and wound around a distal portion of the shaft (12); **characterized in that**
the first coil body (20, 120, 220) is formed by helically twisting at least one twisted wire (22, 122) which is formed by twisting a plurality of single strands (21) together, and
a joint portion (35, 135, 235, 335, 435, 535) impregnated into the at least one twisted wire (22, 122) is disposed in an intermediate part located between a distal end and a proximal end of the first coil body (20, 120, 220) without contacting the shaft (12), so as to join adj oining turns of the first coil body (20, 120, 220) with each other.

2. The guide wire (100) of Claim 1, wherein a pitch of the first coil body (120) increases from the proximal end to the distal end.

3. The guide wire (200, 300, 400, 500) of Claim 1 or 2, wherein the first coil body (220) is formed by helically twisting a plurality of twisted wires (22).

4. The guide wire (300, 400, 500) of any one of Claims 1 to 3, wherein a second coil body (360, 460, 560) is disposed inside the first coil body (220) and is wound around a distal portion of the shaft (12) and the joint portion (335, 435, 535) joins the first coil body (220) and the second coil body (360, 460, 560) without contacting the shaft (12).

5. The guide wire (500) of Claim 4, wherein the second coil body (560) is formed by helically twisting at least one twisted wire (562) which is formed by twisting a plurality of single strands (561) together.

6. The guide wire (500) of Claim 5, wherein the second coil body (560) is formed by helically twisting a plurality of twisted wires (562).

7. The guide wire (300) of Claim 4, wherein the second coil body (360) is formed by helically twisting a single strand (361).

8. The guide wire (400) of Claim 4, wherein the second coil body (460) is formed by twisting a plurality of single strands (461) together.

9. The guide wire (10, 100, 200, 300, 400, 500) of any one of Claims 1 to 8, wherein the joint portion (35, 135, 235, 335, 435, 535) is formed by impregnating resin material into the at least one twisting wire (22, 122).
